(19) Europäisches Patentamt
European Patent Office
Office européen des brevets

(11) Publication number: **0 165 775**
**A1**

# (12) EUROPEAN PATENT APPLICATION

(21) Application number: 85304226.5

(22) Date of filing: 13.06.85

(51) Int. Cl.⁴: C 07 D 249/08
C 07 D 233/68, A 61 K 31/41
A 61 K 31/415, A 01 N 43/653
A 01 N 43/50

(30) Priority: 21.06.84 GB 8415889
06.07.84 GB 8417315

(43) Date of publication of application:
27.12.85 Bulletin 85/52

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Gravestock, Michael Barry
72 Moss Lane
Bramhall Stockport Cheshire(GB)

(74) Representative: Atkinson, John David et al,
Imperial Chemical Industries PLC Legal Department:
Patents PO Box 6 Bessemer Road
Welwyn Garden City Hertfordshire, AL7 1HD(GB)

## (54) Antifungal azole compounds.

(57) A 1-azolylmethyl-2-halogenoazolyl-1-(optionally-substituted phenyl)ethanol, wherein the azolyl groups are imidazolyl or 1,2,4-triazolyl and the optional substituent in the phenyl group is halogen, cyano, alkyl, alkoxy, halogenoalkyl, halogenoalkoxy, alkylsulphonyl, alkylamino, dialkylamino, phenyl, halogenophenyl, phenoxy or halogenophenoxy, processes for its manufacture and antifungal compositions containing said compound.

EP 0 165 775 A1

SUBSTANTIVE SPECIFICATION

TITLE: ANTIFUNGAL AZOLE COMPOUNDS

This invention relates to novel antifungal azoles, and in particular it relates to azolyl-propanol derivatives, to a process for preparing them, to pharmaceutical, veterinary and plant antifungal and plant growth regulating compositions containing them, to processes for controlling fungal infections of plants and to processes for regulating plant growth.

European Patent Application Number 81302146.6 discloses azolyl-propanol derivatives of the formula I wherein R is alkyl, cycloalkyl, aryl or aralkyl, any of which may be optionally substituted, and $A^1$ and $A^2$ are imidazol-1-yl or 1,2,4-triazol-1-yl radicals, and their acid addition salts, metal complexes, ethers and esters, and describes the use of such compounds as pharmaceutical and agricultural antifungals, and as plant growth regulators.

United Kingdom Patent Application Number 2,099,818A describes particularly the compound of the formula I wherein R is a 2,4-difluorophenyl radical, and $A^1$ and $A^2$ are 1,2,4-triazol-1-yl radicals and its pharmaceutical and veterinary antifungal utility.

According to the present invention there is provided a compound of the formula II wherein X and Y, which may be the same or different, are each a methylidyne, halogenomethylidyne or nitrilo radical, $R^1$ is a phenyl radical optionally bearing one or more substituents selected from halogen, cyano, 1-6C alkyl, alkoxy, halogenoalkyl, halogenoalkoxy, alkylsulphonyl and alkylamino radicals, di(1-6C alkyl)amino, phenyl, halogenophenyl, phenoxy and halogenophenoxy radicals, at

least one of $R^2$ and $R^3$ is a fluorine, chlorine or bromine atom, and the other of $R^2$ and $R^3$, if any, is hydrogen.

When X or Y is a halogenomethylidyne radical, the halogen is preferably chlorine or bromine, but it is most preferred that X and Y are each a nitrilo radical, so that the azole rings are each a 1,2,4-triazole ring.

Suitable optional substituents in $R^1$ are, for example, a halogen atom, for example a fluorine, chlorine, bromine or iodine atom, a cyano radical, a 1-6C alkyl, alkoxy, halogenoalkyl or halogenoalkoxy radical, for example a methyl, propyl, hexyl, methoxy, <u>tert</u>-butoxy, hexyloxy, trichloromethyl, trifluoromethyl, trifluoromethoxy or 2,2,2-trifluoroethyl radical, a 1-6C alkylamino or di(1-6C alkyl)amino radical, for example a methylamino, hexylamino, dimethylamino or diethylamino radical, a 1-6C alkylsulphonyl radical, for example a mesyl radical, a halogenophenyl radical, for example a chlorophenyl, bromophenyl or fluorophenyl radical, such as a 4-chlorophenyl radical, and a halogenophenoxy radical, for example a chlorophenoxy, bromophenoxy or fluorophenoxy radical, such as a 4-chlorophenoxy radical. Up to five such optional substituents may be present, but mono- and di-substituted such radicals are preferred.

Specific preferred values for $R^1$ are the 4-chloro-, 4-fluoro-, 4-cyano-, 4-trifluoromethyl-, 4-trifluoromethoxy-, 2,4-dichloro-, 2,4-difluoro- 2-chloro-4-fluoro-, 4-chloro-2-fluoro-, 2-chloro-4-methoxy- and 2-fluoro-4-trifluoromethyl-phenyl, biphenyl, 4'-chloro-4-biphenylyl, 4-phenoxyphenyl and 4-(4-chlorophenoxy)phenyl radicals.

A preferred group of compounds of the invention comprises those wherein $R^2$ is a chlorine or bromine atom and $R^3$ is a hydrogen atom.

Particular preferred compounds of the invention

are 1-(3-bromo-1,2,4-triazol-1-yl)-2-(2,4-difluorophenyl)-3-(1,2,4-triazol-1-yl)-2-propanol, 1-(3-bromo-1,2,4-triazol-1-yl)-2-(4-chlorophenyl)-3-(1,2,4-triazol-1-yl)-2- propanol, 2-(4-chlorophenyl)-1-(3-chloro-1,2,4-triazol-1-yl)-3-(1,2,4-triazol-1-yl)-2-propanol, 1-(3-chloro-1,2,4-triazol-1-yl)-2-(2-fluoro-4-trifluoromethylphenyl) 3-(1,2,4-triazol-1-yl)-2-propanol, 1-(3-chloro-1,2,4-triazol-1-yl)-2-(2,4-dichlorophenyl)-3-(1,2,4-triazol-1-yl)-2-propanol, 1-(3-bromo-1,2,4-triazol-1-yl)-2-(2-chloro-4-fluorophenyl)-3-(1,2,4-triazol-1-yl)-2-propanol and 1-(3-bromo-1,2,4-triazol-1-yl)-2-(4-chloro -2-fluorophenyl)-3-(1,2,4-triazol-1-yl)-2-propanol.

It will be understood that, since the carbon atom bearing substituents $R^1$ and hydroxy maybe asymmetrically substituted, the compounds of the invention may exist in racemic or optically-active forms. It is common general knowledge in the art how such forms may be separated and isolated, and their antifungal properties determined.

The compounds of the formula II may be prepared by methods known generally for the manufacture of similar compounds. Thus, the following processes are provided as further features of this invention, wherein X,Y,$R^1$, $R^2$ and $R^3$ have the meanings defined above, unless otherwise stated:-

(a) the reaction of an epoxide of the formula III wherein $R^4$ is a radical of the formula IV, either as such or formed <u>in situ</u>, with an azole of the formula V in the presence of a strong base; or

(b) the reaction of a ketone of the formula VI with either a Grignard reagent, $R^1$ MgHal, wherein Hal is a halogen or an aryl lithium derivative, $R^1$Li; or

(c) the reaction of a ketone of the formula VII wherein

$R^4$ is a radical of the formula IV with a reagent of the formula VIII wherein $R^5$ is a radical of the formula IX and Q is a triphenylphosphine halide ($Hal^-.Ph_3P^+-$) or dialkyl phosphite [$(R^9O)_2PO-$, wherein $R^9$ is 1-6C lower alkyl] radical, which reagent may be preformed or formed in situ; or

(d) the chlorination or bromination of a compound of the formula II wherein $R^2$ and $R^3$ are each a hydrogen atom, and X and Y are each nitrilo.

The epoxide of the formula III which is used as a starting material in the above process (a) may be obtained by bromination of an appropriately-substituted acetophenone X to give the corresponding phenacyl bromide XI, which is then reacted with an appropriate azole, IV, for example in acetonitrile in thepresence of triethylamine at room temperature, to form an azolyl ketone of the formula VII. The azolyl ketone VII is then reacted with dimethyl sulphonium methylide or dimethyl oxosulphonium methylide to give the required epoxide starting material.

The ketone of the formula VI, used as the starting material in process (b) above, may be obtained by reacting 1,3-dichloro-2-propanone with an azole IV or V (for symmetrical starting materials) or successively with two different azoles, IV and V (for unsymmetrical starting materials of the formula VI).

The preparation of the ketone VII used as starting material in process (c), is described above as an intermediate step in the manufacture of the epoxide starting material III.

Process (d) is preferably carried out by reacting the unsubstituted azole starting material with N-chloro- or N-bromo-succinimide in an inert solvent, for example in chloroform or carbon tetrachloride

at reflux temperature for about 2 days, when the major product is the compound of the formula II bearing a halogen substituent in the 5-position of one 1,2,4-triazole ring only.

The Wittig reagent of the formula VIII used as starting material in the above process (c), may be manufactured by reacting 1-chloromethyl-1,2,4-triazole with either triphenylphosphine, as described in European Patent Publication No. 60222, or with potassium diethyl phosphite.

As indicated above, the compounds of the invention possess antifungal properties which make them useful in the treatment of candidosis and human dermatophyte infections.

This antifungal activity against Candida albicans, a causative fungus of candidosis, and Trichophyton mentagrophytes, var. quinkeanum, a causative fungus of ringworm, was demonstrated as follows:-

Female mice of around 30g. weight are injected sub-cutaneously on a Friday with 0.5mg. of oestradiol benzoate. The following Monday (day 0) they are clipped on the back and then dosed orally with test compounds. They are then inoculated with Candida albicans in the vagina and Trichophyton mentagrophytes var. quinkeanum on the back, and then given a second dose of the same compound. Dosing is repeated once daily on days 1-4. On day 7 skin lesions are scored visually and vaginal samples taken for culture on agar. Groups of 5 mice are used and compounds are dosed initially at a level of 250mg./kg. The dose is then reduced sequentially until a minimum effective dose (MED) is found. For example, the MED for 1-(3-bromo-1,2,4-triazol-2-yl)-2-(2,4-difluorophenyl)-3-(1,2,4-triazol-2-yl)propan-2-ol in this test was 2.5 mg. per kg., and no overt toxicity was

seen at this MED.

Thus, according to a further feature of the invention there is provided a pharmaceutical or veterinary antifungal composition which comprises an antifungally effective amount of compound of the formula II together with a pharmaceutically or veterinarily acceptable diluent or carrier.

The composition of the invention may be in a conventional pharmaceutical form suitable for oral administration, for example a tablet, a capsule, an emulsion or an aqueous or oily solution or suspension, or suitable for topical application, for example a cream, ointment or gel. The composition may contain conventional pharmaceutical excipients, and may be manufactured by conventional pharmaceutical techniques.

Preferred pharmaceutical or veterinary compositions of the invention are compositions suitable for oral administration, and particularly tablets and capsules containing from 1 to 100, preferably 5 to 50mg. of a compound of the invention.

The compounds of the invention also possess antifungal properties which are useful in combatting a wide variety of plant fungal diseases.

The compounds can move acropetally when applied to the plant tissue, and can also be volatile enough to be active in the vapour phase against fungi on the plant.

The compounds may be used as such for plant fungicidal purposes but are more conveniently formulated into compositions for such usage. The invention thus provides also a plant fungicidal composition comprising a compound of general formula II and a non-pharmaceutical carrier or diluent.

The invention also provides a method of

combatting fungal diseases in a plant, which method comprising applying to the plant, to seed of the plant or to the locus of the plant or seed a compound of the formula II.

The compound can be applied in a number of ways, for example it can be applied, formulated or unformulated, directly to the foliage of a plant, to seeds or to the medium in which plants are growing or are to be planted, or it can be sprayed on, dusted on or applied as a cream or paste formulation, or it can be applied as a vapour. Application can be to any part of the plant, bush or tree, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged, and the choice of a suitable conventional composition, and the method by which such a composition may be manufactured, are apparent to those skilled in the art.

The plant fungicidal compositions of this invention can comprise also other compound(s) having biological activity, e.g. compounds having similar or complementary fungicidal activity or compounds having plant growth regulating, hericidal or insecticidal activity.

by the following Examples, in which temperatures are given in degrees Celsius.

Example 1

A solution of 3-bromo-1-(2,4-difluorophenacyl)-1,2,4-triazole (4.65 g.), trimethyloxosulphonium iodide (4.0 g.), 1,2,4-triazole (1.5 g.) and potassium hydroxide (2.1 g.) in tert- butyl alcohol (75 ml.) was heated under reflux for 6 hours, cooled and partitioned between 0.1N hydrochloric acid and ethyl acetate. The ethyl acetate layer was separated, washed with 0.1N hydrochloric acid, then with water and then with saturated brine, and dried by filtering through anhydrous sodium sulphate. The solvent was evaporated, and the residual red gum was purified by medium pressure liquid chromatography (mplc), on silica gel with ethyl acetate as eluant, to give a foam which was dissolved in ethyl acetate and precipitated with diethyl ether to give 1-(3-bromo-1,2,4-triazol-1-yl)-2-(2,4-difluorophenyl)-3-(1,2,4-triazol-1-yl)propan-2-ol, m.p. 91-94°

The 3-bromo-1-(2,4-difluorophenacyl)-1,2,4-triazole used as the starting material in the above process may be obtained as follows:-

2,4-Difluoroacetophenone (15.6 g.) was stirred in chloroform (125 ml.) under illumination from a 250 W. electric lamp, while a solution of bromine 5.25 ml.) in chloroform (25 ml.) was added over 30 minutes after initial decolorisation had begun. The reaction mixture was evaporated to dryness under reduced pressure, then re-evaporated from chloroform twice more to remove any remaining hydrogen bromide, to leave 2,4-difluorophenacyl bromide as a pale yellow oil which was used in the next stage without further purification.

2,4-Difluorophenacyl bromide (12 g.) was stirred in acetonitrile (100 ml.) with 3-bromotriazole (7.35 g.) and triethylamine (10 ml.) at room temperature for 1 hour. The solution became yellow and triethylamine hydrobromide separated. After 1 hour, the total reaction mixture was partitioned between 0.1N hydrochloric acid and ethyl acetate, and the ethyl acetate layer was separated and washed twice with 0.1N hydrochloric acid and once with saturated brine. The solution was filtered through anhydrous sodium sulphate and evaporated to dryness under reduced pressure, to give a red gum, which was purified by m.p.l.c. on silica gel, eluting with ethyl acetate. The product was an oil which solidified on standing, and was crystallised from diethyl ether to give 3-bromo-1-(2,4-difluorophenacyl)-1,2,4-triazole, m.p. 101-104°.

**Examples 2-15**

The process described in Example 1 was repeated, using the appropriately substituted phenacyl triazole or imidazole in place of 3-bromo-1-(2,4-difluorophenacyl)-1,2,4- triazole, to give the following compounds of the formula II ($R^3$ = hydrogen, Y = nitrilo):-

| Ex. | $R^1$ | X | $R^2$ | M.P |
|---|---|---|---|---|
| 2 | 2,4-difluorophenyl | N | Cl | 88-90 |
| 3 | 2,4-difluorophenyl | CH | Cl | (a) |
| 4 | 4-trifluoromethylphenyl | N | Br | (b) |
| 5 | 4-trifluoromethylphenyl | N | Cl | 120-122 |
| 6 | 4-trifluoromethylphenyl | CH | Br | 151-152 |
| 7 | 4-trifluoromethylphenyl | CH | Cl | 144-146(c) |
| 8 | 4-chlorophenyl | N | Br | 134-137 |
| 9 | 4-chlorophenyl | N | Cl | 115-118 |
| 10 | 2,4-dichlorophenyl | N | Br | 164-167 |
| 11 | 2,4-dichlorophenyl | N | Cl | 170-173 |
| 12 | 4-fluorophenyl | N | Br | 114-117 |
| 13 | 4-fluorophenyl | N | Cl | 127-130 |
| 14 | 4-trifluoromethoxyphenyl | N | Cl | 118-120 |
| 15 | 2-fluoro-4-trifluoromethyl--phenyl | N | Cl | 144-145 |

(a) Nmr in $CDCl_3$: 4.30 (q, 2H), 4.6 (q, 2H), 5.65 (br s, 1H), 6.80 (m, 2H), 6.80 (d, 1H), 7.23 (d, 1H), 7.35 (m, 1H), 7.82 (s, 1H), 7.92 (s, 1H).

(b) Nmr in $CDCl_3$; 4.33 (q, 4H), 5.38 (br s, 1H), 7.30 (q, 4H), 7.66 (s, 1H), 7.76 (s, 2H).

(c) contained about 30% of the 5-chloro isomer.

The appropriately substituted phenacyl azoles used as starting materials for the manufacture of the above products were prepared by the process described in the second part of Example 1, using the appropriately substituted acetophenone in place of 2,4-difluoroacetophenone.

**Example 16**

1,3-Bis(1,2,4-triazol-1-yl)-2-(4-trifluoromethylphenyl)propan-2-ol (3.4 g.), carbon tetrachloride (50 ml.), N-chlorosuccinimide (1.5 g.) and dibenzoyl peroxide (100 mg.) were stirred together and heated under reflux for 48 hours. The mixture was cooled and partitioned between water and ethyl acetate, and the organic layer was separated, washed with water and then saturated brine, and dried. The solvent was evaporated under reduced pressure, and the residual pale yellow gum was purified by m.p.l.c. on silica gel, eluting with ethyl acetate, to give a gum which would not crystallise. (N.m.r. in deuteriochloroform: 4.40 (quartet, 2H), 4.65 (quartet, 2H), 7.43 (quartet, 4H) 7.78-7.92-8.16 (singlets, 3H) p.p.m.

**Examples 17-19**

The process described in Example 16 was repeated, using the appropriately substituted 1,3-bis(1,2,4-triazol-1-yl)-2-phenylpropan-2-ol as starting material, to give the following compounds of the formula II (X=Y=N, $R^2$=H):-

| Ex | $R^1$ | $R^3$ | M.p. |
|---|---|---|---|
| 17 | 2,4-difluorophenyl | Br | 112-115 |
| 18 | 4-trifluoromethylphenyl | Br | 115-118 |
| 19 | 4-chlorophenyl | Br | (a) |

(a) Nmr in $CDCl_3$; 4.45 (q, 2H), 4.64 (d, 2H), 5.50 (br s, 1H), 7.23 (s, 4H), 7.80, 7.86 and 8.12 (3 x s, 3H).

Examples 20-26

The process described in Example 1 was repeated, using the appropriately substituted phenacyl triazole in place of 3-bromo-1-(2,4-difluorophenacyl)-1,2,4-triazole, to give the following compounds of the formula II ($R^2$=bromine, $R^3$=hydrogen, X= Y=nitrilo):-

| Ex | $R^1$ | M.p (°) |
|---|---|---|
| 20 | 2-Cl-4-F-phenyl | 108-111 |
| 21 | 4-Cl-2-F-phenyl | 145-148 |
| 22 | 4-biphenylyl | 183-186 |
| 23 | 4'-Cl-4-biphenylyl | (a) |
| 24 | 4-phenoxyphenyl | 93-96 (b) |
| 25 | 4-(4-Cl-phenoxy)phenyl | (b),(c) |
| 26 | 2-Cl-4-MeO-phenyl | 122-125 |

(a) Nmr in $CDCl_3$: 4.50 (s, 2H), 4.55 (q, 2H), 7.30 (s, 1H), 7.42 (m, 8H), 7.91 (s, 1H), 7.96 (s, 1H), 7.78 (s, 1H).

(b) Prepared from the corresponding known phenacyl chlorides as intermediates.

(c) Nmr in $CDCl_3$: 4.48 (s, 2H), 4.52 (q, 2H), 5.22 (br s, 1H), 6.98 (m, 4H), 7.25 (m, 4H), 7.90 (s, 1H), 7.94 (s, 1H), 7.95 (s, 1H).

0165775

$A^1-CH_2-CR(OH)-CH_2-A^2$ I

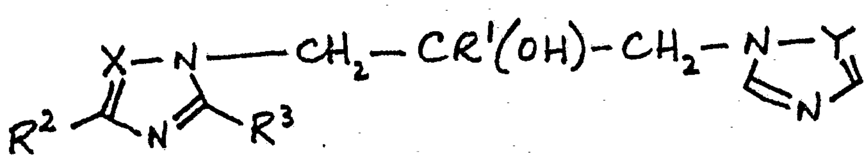

II

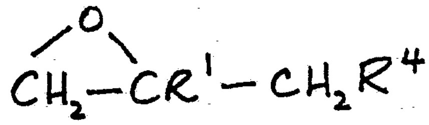

III

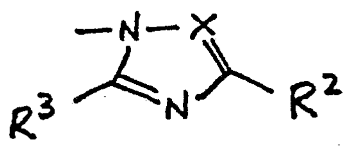

IV

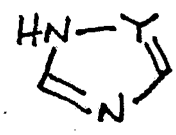

V

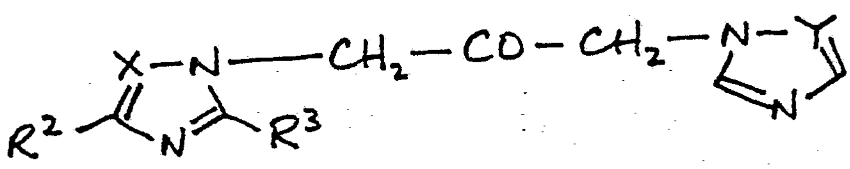

VI

$R^4CH_2COR^1$ VII

$R^5CH_2-Q$ VIII

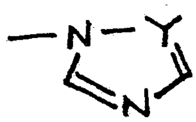

IX

$R^1COCH_3 \longrightarrow R^1COCH_2Br \longrightarrow VII \longrightarrow III$

X XI

## CLAIMS

1. A compound of the formula II wherein X and Y, which may be the same or different, are each a methylidyne, halogenomethylidyne or nitrilo radical, $R^1$ is a phenyl radical optionally bearing one or more substituents selected from halogen, cyano, 1-6C alkyl, alkoxy, halogenoalkyl, halogenoalkoxy, alkylsulphonyl and alkylamino radicals, di(1-6C alkyl)amino, phenyl, halogenophenyl, phenoxy and halogenophenoxy radicals, at least one of $R^2$ and $R^3$ is a fluorine, chlorine or bromine atom, and the other of $R^2$ and $R^3$, if any, is hydrogen.

2. A compound as claimed in claim 2 wherein X or Y is a chloromethylidyne or bromomethylidyne radical.

3. A compound as claimed in claim 1 wherein X and Y are each a nitrilo radical and $R^1$ is phenyl optionally bearing one or more substituents selected from fluorine, chlorine, bromine, iodine, cyano, methyl, propyl, hexyl, methoxy, <u>tert</u>-butoxy, hexyloxy, trichloromethyl, trifluoromethyl, trifluoromethoxy, 2,2,2-trifluoro-ethoxy, methylamino, hexylamino, dimethylamino, diethylamino, mesyl, biphenylyl, 4'-chloro-4-biphenylyl, 4-phenoxyphenyl and 4-(4-chlorophenoxy)phenyl.

4. A compound as claimed in claim 3 wherein $R^1$ is a 4-chloro-, 4-fluoro-, 4-cyano-, 4-trifluoromethyl-, 4-trifluoromethoxy-, 2,4-dichloro-, 2,4-difluoro-, 2-chloro-4-fluoro-, 4-chloro-2-fluoro-, 2-chloro-4-methoxy-, 2-fluoro-4-trifluoromethyl-phenyl, biphenylyl, 4'-chloro-4-biphenylyl, 4-phenoxyphenyl or 4-(4-chlorophenoxy)phenyl radical.

5. A compound as claimed in claim 4 wherein $R^2$ is chlorine or bromine and $R^3$ is hydrogen.

6. A compound as claimed in claim 1 which is 1-(3-bromo-1,2,4-triazol-1-yl)-2-(2,4-difluorophenyl)-

3-(1,2,4-triazol-1-yl)-2-propanol, 1-(3-bromo-1,2,4-triazol-1-yl)-2-(4-chlorophenyl)-3-(1,2,4-triazol-1-yl)-2-propanol, 2-(4-chlorophenyl)-1-(3-chloro-1,2,4-triazol-1-yl)-3-(1,2,4-triazol-1-yl)-2-propanol, 1-(3-chloro-1,2,4-triazol-1-yl)-2-(2-fluoro-4-trifluoromethylphenyl)-3-(1,2,4-triazol-1-yl)-2-propanol, 1-(3-chloro-1,2,4-triazol-1-yl)-2-(2,4-dichlorophenyl)-3-(1,2,4-triazol-1-yl)-2-propanol, 1-(3-bromo-1,2,4-triazol-1-yl)-2-(2-chloro-4-fluorophenyl)-3-(1,2,4-triazol-1-yl)-2-propanol or 1-(3-bromo-1,2,4-triazol-1-yl)-2-(4-chloro-2-fluorophenyl)-3-(1,2,4-triazol-1-yl)-2-propanol.

7. A process for the manufacture of a compound as claimed in claim 1 which comprises:

(a) the reaction of an epoxide of form III wherein $R^4$ is a radical of the formula IV, either as such or formed <u>in situ</u>, with an azole of the formula V in the presence of a strong base; or

(b) the reaction of a ketone of the formula VI with either a Grignard reagent, $R^1$ MgHal, wherein Hal is a halogen or an aryl lithium derivative, $R^1Li$; or

(c) the reaction of a ketone of the formula VII wherein $R^4$ is a radical of the formula IV with a reagent of the formula VIII wherein $R^5$ is a radical of the formula IX and Q is a triphenylphosphine halide ($Hal^-.ph_3P^+-$) or dialkyl phosphite [$R^9O)_2PO-$, wherein $R^9$ is 1-6C lower alkyl] radical, which reagent may be preformed or formed <u>in situ</u>; or

(d) the chlorination or bromination of a compound of the formula II wherein $R^2$ and $R^3$ are each a hydrogen atom, and X and Y are each nitrilo;

wherein X, Y, $R^1$, $R^2$ and $R^3$ have the meanings defined in claim 1, unless otherwise stated.

8. A pharmaceutical or veterinary antifungal composition which comprises an antifungally effective

0165775

amount of compound of the formula II together with a pharmaceutically or veterinarily acceptable diluent or carrier.

9. The use of a compound as claimed in claim 1 for the manufacture of an antifungal medicament.

DS33159
SD10
JDA/KEB: 21 May 85

CLAIMS FOR AUSTRIA

1. A process for the manufacture of a compound of the formula II wherein X and Y, which may be the same or different, are each a methylidyne, halogenomethylidyne or nitrilo radical, $R^1$ is a phenyl radical optionally bearing one or more substituents selected from halogen, cyano, 1-6C alkyl, alkoxy, halogenoalkyl, halogenoalkoxy, alkylsulphonyl and alkylamino radicals, di(1-6C alkyl)amino, phenyl, halogenophenyl, phenoxy and halogenophenoxy radicals, at least one of $R^2$ and $R^3$ is a fluorine, chlorine or bromine atom, and the other of $R^2$ and $R^3$, if any, is hydrogen, which comprises:-

(a) the reaction of an epoxide of form III wherein $R^4$ is a radical of the formula IV, either as such or formed <u>in situ</u>, with an azole of the formula V in the presence of a strong base; or

(b) the reaction of a ketone of the formula VI with either a Grignard reagent, $R^1$ MgHal, wherein Hal is a halogen or an aryl lithium derivative, $R^1Li$; or

(c) the reaction of a ketone of the formula VII wherein $R^4$ is a radical of the formula IV with a reagent of the formula VIII wherein $R^5$ is a radical of the formula IX and Q is a triphenylphosphine halide ($Hal^-,ph_3P^+-$) or dialkyl phosphite [$R^9O)_2PO-$, wherein $R^9$ is 1-6C lower alkyl] radical, which reagent may be preformed or formed <u>in situ</u>; or

(d) the chlorination or bromination of a compound of the formula II wherein $R^2$ and $R^3$ are each a hydrogen atom, and X and Y are each nitrilo;

wherein X, Y, $R^1$, and $R^2$ and $R^3$ have the meanings defined in claim 1, unless otherwise stated.

DS33159/OEE
SD10
JDA/KEB: 22 May 85

JOHN DAVID ATKINSON
AUTHORISED REPRESENTATIVE
General Authorisation No. 97

European Patent Office

# EUROPEAN SEARCH REPORT

0165775
Application number

EP 85304226.5

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl.4) |
| X | EP - A2 - 0 096 569 (PFIZER)<br>* Formula I; examples 1,6,8 *<br>-- | 1,8 | C 07 D 249/08<br>C 07 D 233/68<br>A 61 K 31/41<br>A 61 K 31/415<br>A 01 N 43/653<br>A 01 N 43/50 |
| A | EP - A1 - 0 105 166 (BAYER)<br>* Formula I *<br>-- | 1 | |
| A | EP - A1 - 0 040 761 (HOFFMANN - LA ROCHE)<br>* Claim 2 *<br>-- | 1 | |
| A | CHEMICAL ABSTRACTS, vol. 90, no. 23, June 4, 1979, Columbus, Ohio, USA<br>SMITH, LEVERETT R.; WILKINSON, C.F. "Inhibition of microsomal oxidation by some bis(imidazolyl) derivatives."<br>page 186, column 2, abstract no. 181 494r<br>& Biochem. Pharmacol. 1978, 27(9), 1383-4<br>---- | 1 | TECHNICAL FIELDS SEARCHED (Int. Cl.4)<br>C 07 D 249/00<br>C 07 D 233/00 |

The present search report has been drawn up for all claims

| Place of search | Date of completion of the search | Examiner |
|---|---|---|
| VIENNA | 07-08-1985 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503.03.82